(19) Europäisches Patentamt
European Patent Office
Office européen des brevets

(11) Numéro de publication: **0 395 527 B1**

(12) **FASCICULE DE BREVET EUROPEEN**

(45) Date de publication de fascicule du brevet: **14.09.94**

(51) Int. Cl.5: **C07D 487/04**, A61K 31/55, //(C07D487/04,243:00,235:00)

(21) Numéro de dépôt: **90401151.7**

(22) Date de dépôt: **27.04.90**

# (54) Nouvelles imidazobenzodiazépines et leurs sels, procédé de préparation, application à titre de médicaments et compositions les renfermant.

(30) Priorité: **27.04.89 GB 8909700**

(43) Date de publication de la demande:
**31.10.90 Bulletin 90/44**

(45) Mention de la délivrance du brevet:
**14.09.94 Bulletin 94/37**

(84) Etats contractants désignés:
**AT BE CH DE DK ES FR GB GR IT LI LU NL SE**

(56) Documents cités:
**EP-A- 0 027 214**
**EP-A- 0 285 837**
**EP-A- 0 305 298**

(73) Titulaire: **ROUSSEL-UCLAF**
**35, Boulevard des Invalides**
**F-75007 Paris (FR)**

(72) Inventeur: **Hedgecock, Charles John Robert**
**186 High Street,**
**Wootton Bassett**
**Swindon SN4 7B7, Wiltshire (GB)**
Inventeur: **Kuo, Elizabert Anne**
**16, Sandpiper Bridge**
**Covingham**
**Swindon SN3 5DY, Wiltshire (GB)**
Inventeur: **Jones, Stuart Donald**
**1 Walnut Tree Gardens**
**Lydiard Millicent**
**Swindon SN5 9IH, Wiltshire (GB)**

(74) Mandataire: **Vieillefosse, Jean-Claude et al**
**Roussel-Uclaf**
**111, route de Noisy**
**B.P. 9**
**F-93230 Romainville (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention concerne de nouvelles imidazobenzodiazépines et leurs sels, ainsi que le procédé de préparation, l'application à titre de médicaments de ces nouveaux produits et les compositions les renfermant.

L'invention a pour objet de nouvelles imidazobenzodiazépines répondant à la formule générale (I) :

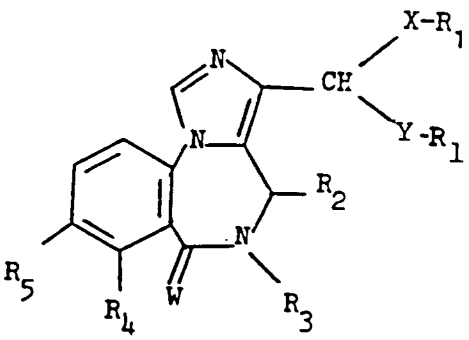

(I)

dans laquelle :

- les substituants $R_1$, représentent un radical alcoyle renfermant de 1 à 3 atomes de carbone ou forment ensemble un pont alkylène renfermant de 2 à 5 atomes de carbone,
- X et Y identiques ou différents représentent un atome d'oxygène ou un atome de soufre,
- $R_2$ représente un atome d'hydrogène, un radical alcoyle renfermant de 1 à 3 atomes de carbone,
- $R_3$ représente un atome d'hydrogène, un radical alcoyle renfermant de 1 à 5 atomes de carbone un groupement phényle un groupement phényle substitué,
- W représente un atome d'oxygène ou un atome de soufre,
- $R_4$ et $R_5$, identiques ou différents, représentent un atome d'hydrogène, un atome d'halogène, un radical hydroxy, un radical alcoyle ou alcoxy renfermant de 1 à 3 atomes de carbone, un groupement trifluorométhyle, étant entendu que lorsque chacun des substituants $R_1$ représente un radical alcoyle, X et Y sont identiques ainsi que leurs sels d'addition avec les acides minéraux ou organiques.

Dans la formule générale (I) et dans ce qui suit :

- par radical alcoyle renfermant de 1 à 3 atomes de carbone, on entend un radical méthyle, éthyle, propyle, isopropyle ;
- par radical alkylène renfermant de 2 à 5 atomes de carbone, on entend de préférence un radical éthylène, triméthylène, tétraméthylène ou pentaméthylène ;
- par radical alcoyle renfermant de 1 à 5 atomes de carbone, on entend de préférence un radical méthyle, éthyle, propyle, isopropyle, butyle, isobutyle, terbutyle ou pentyle ;
- par groupement phényle substitué on entend de préférence un groupement phényle substitué par un atome d'halogène, un radical alcoyle renfermant de 1 à 3 atomes de carbone, un radical alcoxy renfermant de 1 à 3 atomes de carbone, un groupement trifluorométhyle ;
- par atome d'halogène, on entend de préférence un atome de fluor, de chlore ou de brome ;
- par radical alcoxy renfermant de 1 à 3 atomes de carbone, on entend de préférence un radical méthoxy, éthoxy, propoxy ou isopropoxy.

Les sels d'addition avec les acides minéraux ou organiques peuvent être, par exemple, les sels formés avec les acides chlorhydrique, bromhydrique, iodhydrique, nitrique, sulfurique, phosphorique, propionique, formique, benzoïque, maléïque, fumarique, succinique, tartrique, citrique, oxalique, glyoxylique, aspartique, alcanesulfoniques tel que l'acide méthanesulfonique et arylsulfoniques tel que l'acide benzènesulfonique.

Parmi les produits, objet de l'invention, on peut citer notamment les dérivés répondant à la formule (I) ci-dessus, caractérisés en ce que dans ladite formule (I), W représente un atome d'oxygène, $R_2$ et $R_5$ représentent un atome d'hydrogène, $R_4$ représente un atome d'halogène ou un groupement trifluorométhyle et X, Y, $R_1$ et $R_3$ ont la signification déjà indiquée, ainsi que leurs sels d'addition avec les acides minéraux ou organiques.

Parmi ces derniers, on peut citer les dérivés répondant à la formule (I) ci-desus dans laquelle $R_3$ représente un radical méthyle, $R_1$, Y, X, $R_2$, $R_4$, $R_5$ et W ont la signification déjà indiquée, ainsi que leurs sels d'addition avec les acides minéraux ou organiques et notamment :

- la 5,6- dihydro-3-(1,3-dioxalan-2-yl)-5-méthyl-imidazo [1,5-a> [1,4] benzodiazépine-6 (4H)-one ;

- la 5,6-dihydro-3-(1,3-dithiolan-2-yl)-5-méthyl-imidazo [1,5-a] [1,4] benzodiazépine-6 (4H)-one ;
- la 5,6-dihydro 3-(1,3-dioxolan-2-yl) 5-méthyl 7-trifluorométhyl imidazo [1,5-a] [1,4] benzodiazépine 6-(4H)-one ;
- la 5,6-dihydro 5-méthyl 3-(1,3-oxathiolan-2-yl) 7-trifluorométhyl imidazo [1,5-a] [1,4] benzodiazépine 6-(4H)-one ;
- la 5,6-dihydro 5-méthyl 3-(1,3-oxathiolan-2-yl) imidazo [1,5-a] [1,4] benzodiazépine 6(4H)-one ;
- la 5,6-dihydro 7-fluoro 5-méthyl 3-(1,3-oxathiolan-2-yl) imidazo [1,5-a] [1,4] benzodiazépine 6(4H)-one ;

ainsi que leurs sels d'addition avec les acides.

L'invention a également pour objet un procédé de préparation des nouvelles imidazobenzodiazépines telles que définies par la formule (I) ci-dessus, ainsi que de leurs sels, caractérisé en ce que l'on fait réagir un produit de formule (II) :

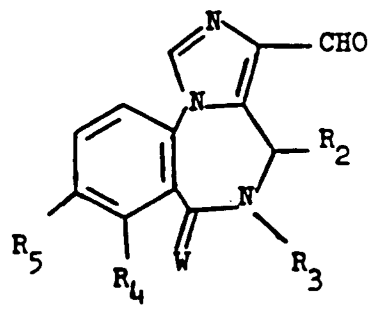

(II)

dans laquelle $R_2$, $R_3$, W, $R_4$ et $R_5$ ont la signification déjà indiquée, soit avec un produit de formule (III) :

H-X-$R_A$ (III)

dans laquelle X a la signification déjà indiquée et $R_A$ représente un radical alcoyle renfermant de 1 à 3 atomes de carbone, pour obtenir un produit de formule ($I_A$) :

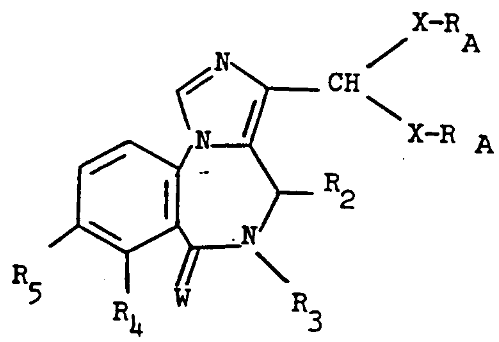

($I_A$)

que l'on peut salifier si désiré, soit avec un produit de formule (IV) :

H-X-$R_B$
H-Y-$R_B$ (IV)

dans laquelle X et Y ont la signification déjà indiquée et les substituants $R_B$ forment ensemble un pont alkylène renfermant de 2 à 5 atomes de carbone, pour obtenir le produit de formule ($I_B$) :

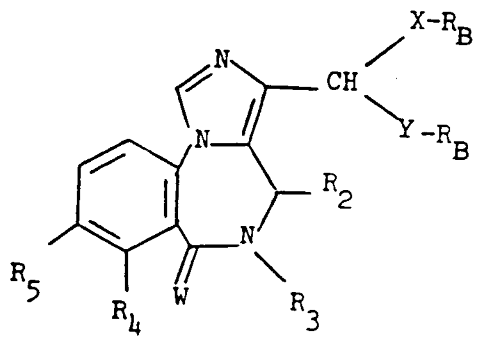

$(I_B)$

que l'on peut salifier si désiré.

Dans des conditions préférentielles de mise en oeuvre de l'invention, le procédé de préparation ci-dessus décrit est caractérisé en ce que la réaction du produit de formule (II) avec le produit de formule (III) ou avec le produit de formule (IV) est effectuée au sein d'un solvant organique anhydre tel que le benzène ou le dichlorométhane.

Lorsque l'on fait réagir un produit de formule (II) avec un produit de formule (III) dans laquelle X représente un atome d'oxygène, ou avec un produit de formule (IV) dans laquelle X et Y représentent chacun un atome d'oxygène, on opère avantageusement au sein d'un solvant organique anhydre tel que le benzène, en présence d'une quantité catalytique d'acide paratoluène sulfonique, au reflux du mélange réactionnel.

Lorsque l'on fait réagir un produit de formule (II) avec un produit de formule (III) dans laquelle X représente un atome de soufre, ou avec un produit de formule (IV) dans laquelle X et Y représentent chacun un atome de soufre, on opère avantageusement au sein d'un solvant organique anhydre tel que le dichlorométhane, en présence d'une quantité catalytique de trifluoroéthérate de bore, à température ambiante.

Les produits de formule (I) présentent un caractère basique. On peut avantageusement préparer les sels d'addition des produits de formule (I) en faisant réagir, en proportions sensiblement stoéchiométriques, un acide minéral ou organique avec lesdits produits de formule (I). Les sels peuvent être préparés sans isoler les bases correspondantes.

Les produits de formule (II) peuvent être préparés selon le procédé indiqué dans la demande de brevet européen publiée sous le n° 0 305 298.

Les produits, objet de la présente invention, possèdent de très intéressantes propriétés pharmacologiques, on note en particulier des propriétés agonistes inverses des benzodiazépines.

Certains produits présentent, en outre, des propriétés tranquilisantes.

Ces propriétés sont illustrées plus loin dans la partie expérimentale.

Ces propriétés justifient l'utilisation des nouvelles imidazobenzodiazépines de formule (I) ainsi que leurs sels pharmaceutiquement acceptables, à titre de médicaments.

La présente invention a aussi également pour objet l'application à titre de médicaments des nouvelles imidazobenzodiazépines telles que définies par la formule générale (I) ainsi que de leurs sels d'addition avec les acides pharmaceutiquement acceptables.

Parmi les médicaments, objet de l'invention, on retient notamment les médicaments caractérisés en ce qu'ils sont constitués par les nouvelles imidazobenzodiazépines répondant à la formule (I) dans laquelle W représente un atome d'oxygène, $R_2$ et $R_5$ représentent chacun un atome d'hydrogène, $R_4$ représente un atome d'halogène ou un groupement trifluorométhyle, X, Y, $R_1$ et $R_3$ ont la signification déjà indiquée ainsi que leurs sels d'addition avec les acides pharmaceutiquement acceptables.

Parmi les médicaments, objet de l'invention, on retient tout particulièrement ceux répondant à la formule (I) ci-dessus dans laquelle $R_3$ représente un radical méthyle, $R_1$, Y, X, $R_2$, $R_4$, $R_5$ et W ont la signification déjà indiquée, ainsi que leurs sels d'addition avec les acides pharmaceutiquement acceptables.

Parmi les médicaments préférés de l'invention, on retient tout particuliérement la

- la 5,6- dihydro-3-(1,3-dioxalan-2-yl)-5-méthyl-imidazo [1,5-a] [1,4] benzodiazépine-6 (4H)-one ;
- la 5,6-dihydro-3-(1,3-dithiolan-2-yl)-5-méthyl-imidazo [1,5-a] [1,4] benzodiazépine-6 (4H)-one ;
- la 5,6-dihydro 3-(1,3-dioxolan-2-yl) 5-méthyl 7-trifluorométhyl imidazo [1,5-a] [1,4] benzodiazépine 6-(4H)-one ;
- la 5,6-dihydro 5-méthyl 3-(1,3-oxathiolan-2-yl) 7-trifluorométhyl imidazo [1,5-a] [1,4] benzodiazépine 6-(4H)-one ;
- la 5,6-dihydro 5-méthyl 3-(1,3-oxathiolan-2-yl) imidazo [1,5-a] [1,4] benzodiazépine 6(4H)-one ;

- la 5,6-dihydro 7-fluoro 5-méthyl 3-(1,3-oxathiolan-2-yl) imidazo [1,5-a] [1,4] benzodiazépine 6(4H)-one ;

ainsi que leurs sels pharmaceutiquement acceptables.

Ces médicaments trouvent, par exemple, leur emploi dans le traitement des troubles de la mémoire, notamment en gériatrie, des troubles de la sénescence cérébrale. Certains produits peuvent également être utilisés dans le traitement de l'obésité ainsi que comme tranquilisant mineur dans le traitement de certaines agitations ou irritabilité et dans certaines formes d'épilepsie.

La dose usuelle variable selon le produit utilisé, le sujet traité et l'affection en cause peut être par exemple, de 0,1 mg à 200 mg par jour, par voie orale.

L'invention a également pour objet les compositions pharmaceutiques qui renferment au moins un dérivé précité ou l'un de ses sels d'addition avec les acides pharmaceutiquement acceptables à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés simples ou dragéifiés, les gélules, les capsules, les granulés, les suppositoires, les préparations injectables ; elles sont préparées selon les méthodes usuelles. Le ou les principes actifs peuvent être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Il va être donné maintenant à titre non limitatif des exemples demise en oeuvre de l'invention.

**Exemple 1 : 5,6-dihydro-3-(1,3-Dioxolan-2-yl)-5-methyl-imidazo [1,5-a] [1,4] benzodiazepine-6 (4H)-one**

On chauffe au reflux pendant 5 heures 100 mg de 5,6-dihydro-5-méthyl-6oxo-4H-imidazo [1,5-a] [1,4] benzodiazépine-3-carboxaldehyde dans 3 $cm^3$ de benzène en présence de 0,1 $cm^3$ d'éthylène glycol et d'une quantité catalytique d'acide paratoluène sulfonique. On élimine le solvant, reprend le résidu dans du chlorure de méthylène, le lave avec une solution aqueuse de carbonate de sodium à 5%. Après séchage, on obtient 95 mg de produit attendu.

Preparation du 5,6-dihydro-5-méthyl-6-oxo-4H-imidazo [1,5a]-[1,4] benzodiazépine-3-carboxaldéhyde

On agite à 0°C pendant 4 heures 1 g de 5,6-dihydro-5-méthyl-6-oxo-4H-imidazo [1,5a] [1,4] benzodiazépin-3-yl-N-méthyl carbohydroxamate de méthyle (préparée comme indiqué dans le brevet européen EP. 88 402166 dans 36 $cm^3$ de tétrahydrofuranne en présence de 96 mg d'hydrure d'aluminium-lithium. On laisse revenir à température ambiante, agite 22 heures puis ajoute 33 mg d'hydrure supplémentaire. Après 4 heures d'agitation, on élimine l'excès d'hydrure par addition d'une solution aqueuse de chlorure de sodium, sèche la phase organique et évapore à sec. Après chromatographie sur silice, on obtient 0,59 g de produit attendu.

**Exemple 2 : 5,6-dihydro-3-(1,3-dithiolan-2-yl)-5-methyl-imidazo [1,5a] [1,4] benzodiazepin-6(4H)-one**

On dissout 1,96 g de 5,6-dihydro-5-méthyl-6-oxo-4H-imidazo [1,5a] [1,4] benzodiazépin-3-carboxaldéhyde dans 25 $cm^3$ de chlorure de méthylène et ajoute 1 $cm^3$ d'éthane 1,2-dithiol et 0,2 $cm^3$ de de trifluoroéthérate de bore. On agite pendant 24 heures à température ambiante, ajoute 0,5 $cm^3$ de dithiol supplémentaire et maintient sous agitation pendant 7 jours. On dilue le milieu réactionnel avec du chlorure de méthylène, lave avec une solution aqueuse à 5% de carbonate de sodium et 5% de soude, sèche et concentre. On obtient une huile que l'on purifie par chromatographie sur silice puis cristallisation dans l'éther. On obtient 1,95 g de produit attendu.

**Exemple 3 : 5,6-dihydro 3-(1,3-dioxolan-2-yl) 5-méthyl 7-trifluorométhyl imidazo [1,5-a] [1,4] benzodiazépin-6 (4H)-one.**

En opérant comme à l'exemple 1, on a obtenu le produit attendu.

**Exemple 4 : 5,6-dihydro 5-méthyl 3-(1,3-oxathiolan-2-yl) 7-trifluorométhyl imidazo [1,5-a] [1,4] benzodiazépine-6 (4H) one.**

A 400 mg de 5,6-dihydro 5-méthyl 6-oxo 7-trifluorométhyl 4H-imidazo [1,5-a] [1,4] benzodiazépine 3-carboxaldéhyde en solution dans 12 $cm^3$ de dichlorométhane et 0,04 $cm^3$ de trifluoroéthérate de bore, on ajoute peu à peu en 5 heures 0,18 $cm^3$ de 2-mercaptoéthanol en solution dans 40 $cm^3$ de dichlorométhane. Après 18 heures d'agitation, on ajoute 0,02 $cm^3$ de trifluoroéthérate supplémentaire, puis agite le milieu réactionnel pendant 24 heures à température ambiante. On extrait avec une solution aqueuse de bicarbonate de sodium, sèche et concentre sous pression réduite. Après chromatographie sur silice, on recueille le produit attendu avec un rendement de 47%.

En opérant de manière analogue, on a préparé les produits des exemples 5 et 6.

**Exemple 5 : 5,6-dihydro 5-méthyl 3-(1,3-oxathiolan-2-yl) imidazo [1,5-a] [1,4] benzodiazépine-6 (4H) one.**

**Exemple 6 : 5,6-dihydro 7-fluoro 5-méthyl 3-(1,3-oxathiolan-2-yl) imidazo [1,5-a] [1,4] benzodiazépine-6 (4H) one.**

Les analyses spectrométriques, les rendements et les résultats de la microanalyse sont donnés dans le tableau I ci-après.

TABLEAU I

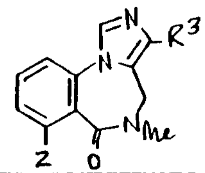

| Exemple | $R_3$ | Z | F°C | Spectre IR $cm^{-1}$ |
|---|---|---|---|---|
| 1 | 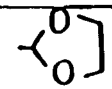 | H | 75.3% 169-170 | 3650-3100(v.br); 1630,1600,1490, 1420,1065,750 |
| 2 | 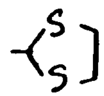 | H | 75.6% 158-160 $Et_2O$ | 3100,2960,2930,1620,1495,1390,1230 940,760 |
| 3 | 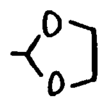 | $CF_3$ | 60% 202 | 3090,2900,1645,1600,1500,1470,1420 1395,1320,1310,1245,1185,1160,1130 1100,1085,1065,1010,955,830,820, 770,720,710,690,655 |
| 4 | 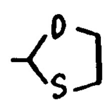 | $CF_3$ | 47% 200-201 | 3090,2900,1645,1495,1470,1425,1390 1320,1190,1155,1130,1095,1080,1055 965,935,825,810,770,730,710,690 and 655$cm^{-1}$ |
| 5 | 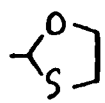 | H | 26% 171-172 | 3550,3380,3090,2920,2860,1635,1595 1580,1490,1430,1390,1290,1245,1220 1150,1060,1010,970,930,905,880,855 820,790,760,720,695 |
| 6 | 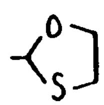 | F | 37% 198-201 | 3105,2940,2860,1635,1590,1580,1490 1470,1435,1400,1290,1255,1220,1150 1055,1045,1020,965,940,915,875,805 770,730,695 and 660 |

TABLEAU I (suite)

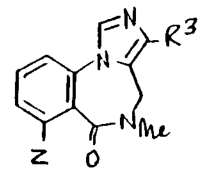

| Exemple | Spectre RMN | | Analyse |
|---|---|---|---|
| 1 | $CDCl_3$ 8.05(1H,dd); 7.87(1H,s); 7.50(3H,m); 5.96(1H,s); 4.50(1H,brd); 4.20(1H,brd); 4.10(4H,m); 3.21(3H,d) | $C_{15}H_{15}N_3O_3$ 285.31 | 63.15 5.30 14.73<br>62.85 5.34 14.61 |
| 2 | $CDCl_3$ 8.02(1H,dd); 7.65(1H,s); 7.35-7.57(3H,m); 5.80(1H,s); 4.60(1H,brs); 4.41(2H,brs); 3.61(2H,m); 3.40(2H,m); 3.28(3H,s) | $C_{15}H_{15}N_3OS_2$ 317.44 | 56.76 4.76 13.24 S:20.21<br>56.82 4.83 13.09 20.16 |
| 3 | δ($CDCl_3$)3.17(3H,s);4.15(4H,mult);4.32(1H,d,J=16Hz); 4.61(1H,d,J=16Hz);5.95(1H,s);7.54(1H,d,J=8Hz); 7.70(1H,trip,J=8Hz);7.83(1H,d,J=8Hz);7.91(1H,s) | $C_{10}H_{14}N_3F_3O_3$ 353.99 | 54.39 4.00 11.89 16.13<br>54.04 4.00 11.80 16.18 |
| 4 | δ($CDCl_3$)3.16 and 3.21(3H,2 singlets);3.31(2H,mult); 3.96(1H,mult);4.33(1H,dd,J=1.7,16Hz);4.60(2H,mult); 6.20 and 6.22(1H,2 singlets);7.73(3H,mult);7.88 and 7.89(1H,2 singlets) | $C_{16}H_{14}N_3F_3O_2S$ 369.99 | 52.03 3.83 11.37 15.43<br>51.98 3.86 11.36 15.27 |
| 5 | 3.33(5H,m);3.96(1H,mult);4.52(3H,mult);6.22(1H,s); 7.38(1H,mult);7.55(2H,mult);7.84(1H,s);8.04(1H,s). | $C_{15}H_{15}N_3O_2S$ 301.36 | Accurate Mass<br>301.0883<br>301.0857 |
| 6 | 3.31(5H,mult);3.97(1H,mult);4.36(1H,mult);4.58(2H,mult); 6.19 and 621(1H,singlets).7.22(2H,mult);7.56(1H,mult); 7.86(1H,s). | $C_{15}H_{14}N_3F_1S_1$ 319.36 | Accurate Mass<br>Calc 319.0789<br>319.0774 |

**Exemple 7 :**

On a préparé des comprimés répondant à la formulation suivante :

| | |
|---|---|
| - Produit de l'exemple 1<br>- Excipient pour un comprimé terminé à | 20 mg<br>150 mg |
| (Détail de l'excipient : lactose, amidon, talc, stéarate de magnésium). | |

**Exemple 8 :**

On a préparé des comprimés répondant à la formulation suivante :

| | |
|---|---|
| - Produit de l'exemple 2<br>- Excipient pour un comprimé terminé à | 20 mg<br>150 mg |
| (Détail de l'excipient : lactose, amidon, talc, stéarate de magnésium). | |

**Exemple 9 :**

On a préparé des comprimés répondant à la formulation suivante :

| | |
|---|---|
| - Produit de l'exemple 4<br>- Excipient pour un comprimé terminé à | 20 mg<br>150 mg |
| (Détail de l'excipient : lactose, amidon, talc, stéarate de magnésium). | |

**Exemple 10 :**

On a préparé des comprimés répondant à la formulation suivante :

| | |
|---|---|
| - Produit de l'exemple 6<br>- Excipient pour un comprimé terminé à | 20 mg<br>150 mg |
| (Détail de l'excipient : lactose, amidon, talc, stéarate de magnésium). | |

**ACTIVITE PHARMACOLOGIOUE**

Test N° 1

L'affinité des composés pour les récepteurs des benzodiazépines a été évaluée en utilisant un radioligand [$^3$H] flunitrazépam et la méthode de Squires et Braestrup (Nature, 1977, 266, 732 modifiée).

Les valeurs indiquées dans le tableau ci-après sont les concentrations nanomolaires (mol x $10^{-9}$) du produit testé qui inhibent dans une proportion de 50% la liaison spécifique de 0,6 nanomoles de [$^3$H] flunitrazépam dans des préparations de membranes de cerveaux antérieurs de rats [($CI_{50}$) en nanomoles].

Test N° 2

L'évaluation de la liaison aux récepteurs des benzodiazépines, in vivo, a été effectuée selon la méthode décrite par Goeders N.E. et Kuhar M.J., (Life Sciences (1985) 37, 345.

TABLEAU II

| Exemple | Test 1 nM | Test 2 ED50 mg/kg ip |
|---|---|---|
| 1 | 1075 | 1,0 |
| 2 | 94 | 7,0 |
| 3 | 73 | 0,09 |
| 4 | 10 | 0,08 |
| 5 | 339 | 0,8 |
| 6 | 36 | 0,22 |

**Revendications**

**Revendications pour les Etats contractants suivants : AT, BE, CH, DE, DK FR, GB, IT, LI, LU, NL, SE**

**1.** Nouvelles imidazobenzodiazépines répondant à la formule générale (I) :

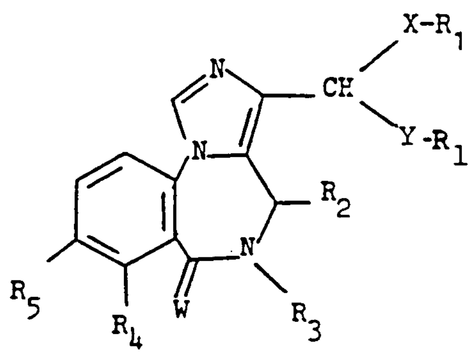

(I)

dans laquelle :

- les substituants $R_1$, représentent un radical alcoyle renfermant de 1 à 3 atomes de carbone ou forment ensemble un pont alkylène renfermant de 2 à 5 atomes de carbone,
- X et Y identiques ou différents représentent un atome d'oxygène ou un atome de soufre,
- $R_2$ représente un atome d'hydrogène, un radical alcoyle renfermant de 1 à 3 atomes de carbone,
- $R_3$ représente un atome d'hydrogène, un radical alcoyle renfermant de 1 à 5 atomes de carbone, un groupement phényle, un groupement phényle substitué,
- W représente un atome d'oxygène ou un atome de soufre,
- $R_4$ et $R_5$, identiques ou différents, représentent un atome d'hydrogène, un atome d'halogène, un radical hydroxy, un radical alcoyle ou alcoxy renfermant de 1 à 3 atomes de carbone, un groupement trifluorométhyle, étant entendu que lorsque chacun des substituants $R_1$ représente un radical alcoyle, X et Y sont identiques ainsi que leurs sels d'addition avec les acides minéraux ou organiques.

**2.** Nouvelles imidazobenzodiazépines telles que définies par la formule (I) de la revendication 1, caractérisés en ce que dans ladite formule (I), W représente un atome d'oxygène, $R_2$ et $R_5$ représentent un atome d'hydrogène, $R_4$ représente un atome d'halogène ou un groupement trifluorométhyle et X, Y, $R_1$ et $R_3$ ont la signification indiquée à la revendication 1, ainsi que leurs sels d'addition avec les acides minéraux ou organiques.

**3.** Nouvelles imidazobendiazépines répondant à la formule (I), selon la revendication 1 ou 2, caractérisées en ce que dans ladite formule (I), $R_3$ représente un radical méthyle, $R_1$, $_{Y,}$ X, $R_2$, $R_4$, $R_5$ et W ont la signification déjà indiquée, ainsi que leurs sels d'addition avec les acides minéraux ou organiques.

**4.** La 5,6- dihydro-3-(1,3-dioxalan-2-yl)-5-méthyl-imidazo [1,5-a] [1,4] benzodiazépine-6 (4H)-one.

**5.** La 5,6-dihydro-3-(1,3-dithiolan-2-yl)-5-méthyl-imidazo [1,5-a] [1,4] benzodiazépine-6 (4H)-one.

**6.** La 5,6-dihydro 3-(1,3-dioxolan-2-yl) 5-méthyl 7-trifluorométhyl imidazo [1,5-a] [1,4] benzodiazépine 6-(4H)-one.

**7.** La 5,6-dihydro 5-méthyl 3-(1,3-oxathiolan-2-yl) 7-trifluorométhyl imidazo [1,5-a] [1,4] benzodiazépine 6-(4H)-one.

**8.** La 5,6-dihydro 5-méthyl 3-(1,3-oxathiolan-2-yl) imidazo [1,5-a] [1,4] benzodiazepine 6(4H)-one.

**9.** La 5,6-dihydro 7-fluoro 5-méthyl 3-(1,3-oxathiolan-2-yl) imidazo [1,5-a] [1,4] benzodiazépine 6(4H)-one.

**10.** Procédé de preparation des nouvelles imidazobenzodiazépines telles que définies par la formule (I) de la revendication 1, ainsi que de leurs sels, caractérisé en ce que l'on fait réagir un produit de formule (II) :

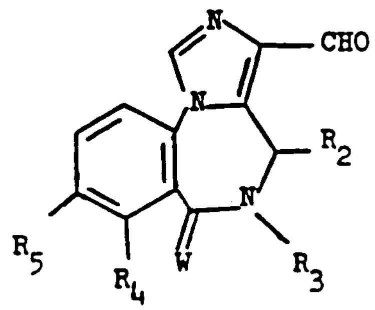

(II)

dans laquelle $R_2$, $R_3$, W, $R_4$ et $R_5$ ont la signification déjà indiquée, soit avec un produit de formule (III) :

$H\text{-}X\text{-}R_A$ (III)

dans laquelle X a la signification déjà indiquée et $R_A$ représente un radical alcoyle renfermant de 1 à 3 atomes de carbone, pour obtenir un produit de formule ($I_A$) :

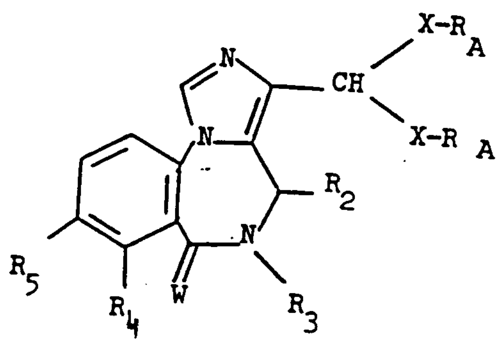

($I_A$)

que l'on peut salifier si désiré, soit avec un produit de formule (IV) :

$H\text{-}X\text{-}R_B$
$H\text{-}Y\text{-}R_B$ (IV)

dans laquelle X et Y ont la signification déjà indiquée et les substituants $R_B$ forment ensemble un pont alkylène renfermant de 2 à 5 atomes de carbone, pour obtenir le produit de formule ($I_B$) :

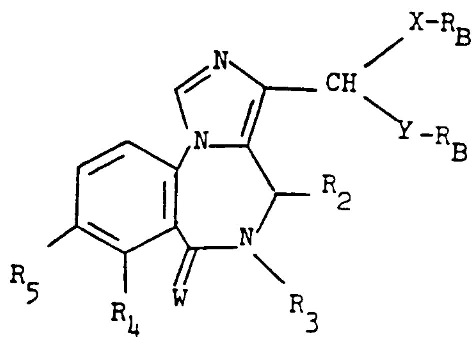

($I_B$)

que l'on peut salifier si désiré.

**11.** Procédé selon la revendication 10, caractérisé en ce que la réaction du produit de formule (II) avec le produit de formule (III) ou avec le produit de formule (IV) est effectuée au sein d'un solvant organique anhydre tel que le benzène ou le dichlorométhane.

**12.** Médicaments, caractérisés en ce qu'ils sont constitués par les nouvelles imidazobenzodiazépines telles que définies par la formule (I) de la revendication 1, ainsi que par leurs sels pharmaceutiquement acceptables.

**13.** Médicaments, caractérisés en ce qu'ils sont constitués par les nouvelles imidazobenzodiazépines telles que définies à l'une quelconque des revendications 2 à 9, ainsi que par leurs sels pharmaceutiquement acceptables.

**14.** Compositions pharmaceutiques, caractérisées en ce qu'elles renferment à titre de principe actif, l'un au moins des médicaments tels que définis à l'une quelconque des revendications 12 ou 13.

**Revendications pour l'Etat contractant suivant : ES**

**1.** Procédé pour préparer des nouvelles imidazobenzodiazépines répondant à la formule générale (I) :

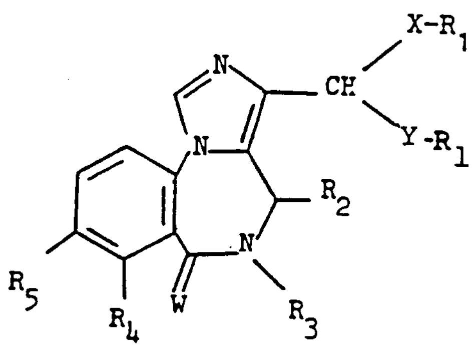

(I)

dans laquelle :

- les substituants $R_1$, représentent un radical alcoyle renfermant de 1 à 3 atomes de carbone ou forment ensemble un pont alkylène renfermant de 2 à 5 atomes de carbone,
- X et Y identiques ou différents représentent un atome d'oxygène ou un atome de soufre,
- $R_2$ représente un atome d'hydrogène, un radical alcoyle renfermant de 1 à 3 atomes de carbone,
- $R_3$ représente un atome d'hydrogène, un radical alcoyle renfermant de 1 à 5 atomes de carbone, un groupement phényle, un groupement phényle substitué,
- W représente un atome d'oxygène ou un atome de soufre,
- $R_4$ et $R_5$, identiques ou différents, représentent un atome d'hydrogène, un atome d'halogène, un radical hydroxy, un radical alcoyle ou alcoxy renfermant de 1 à 3 atomes de carbone, un groupement trifluorométhyle, étant entendu que lorsque chacun des substituants $R_1$ représente un radical alcoyle, X et Y sont identiques ainsi que leurs sels d'addition avec les acides minéraux

ou organiques, caractérisé en ce que l'on fait réagir un produit de formule (II) :

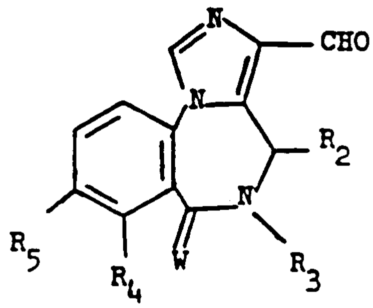

(II)

dans laquelle $R_2$, $R_3$, W, $R_4$ et $R_5$ ont la signification déjà indiquée, soit avec un produit de formule (III) :

$H\text{-}X\text{-}R_A$ (III)

dans laquelle X a la signification déjà indiquée et $R_A$ représente un radical alcoyle renfermant de 1 à 3 atomes de carbone, pour obtenir un produit de formule ($I_A$) :

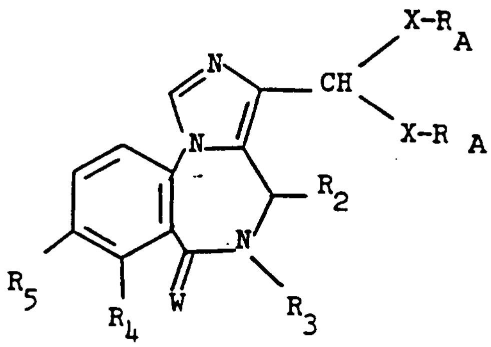

($I_A$)

que l'on peut salifier si désiré, soit avec un produit de formule (IV) :

$H\text{-}X\text{-}R_B$
$H\text{-}Y\text{-}R_B$ (IV)

dans laquelle X et Y ont la signification déjà indiquée et les substituants $R_B$ forment ensemble un pont alkylène renfermant de 2 à 5 atomes de carbone, pour obtenir le produit de formule ($I_B$) :

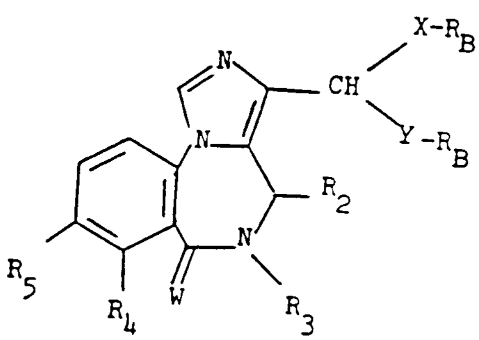

($I_B$)

que l'on peut salifier si désiré.

2. Procédé selon la revendication 1, caractérisé en ce que la réaction du produit de formule (II) avec le produit de formule (III) ou avec le produit de formule (IV) est effectuée au sein d'un solvant organique anhydre tel que le benzène ou le dichlorométhane.

3. Procédé selon la revendication 1 ou 2, carctérisé en ce que l'on utilise au départ un produit de formule (II) dans laquelle W représente un atome d'oxygène, $R_2$ et $R_5$ représentent un atome d'hydrogène, $R_4$ représente un atome d'halogène ou un groupement trifluorométhyle et $R_3$ a la signification indiquée à la revendication 1.

4. Procédé selon l'une quelconque des revendications 1 à 3, carctérisé en ce que l'on utilise au départ un produit de formule (II) dans laquelle $R_3$ représente un radical méthyle.

**Revendications pour l'Etat contractant suivant : GR**

1. Procédé pour préparer des nouvelles imidazobenzodiazépines répondant à la formule générale (I) :

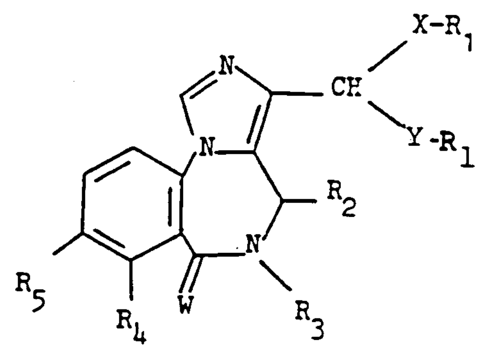

(I)

dans laquelle :
- les substituants $R_1$, représentent un radical alcoyle renfermant de 1 à 3 atomes de carbone ou forment ensemble un pont alkylène renfermant de 2 à 5 atomes de carbone,
- X et Y identiques ou différents représentent un atome d'oxygène ou un atome de soufre,
- $R_2$ représente un atome d'hydrogène, un radical alcoyle renfermant de 1 à 3 atomes de carbone,
- $R_3$ représente un atome d'hydrogène, un radical alcoyle renfermant de 1 à 5 atomes de carbone, un groupement phényle, un groupement phényle substitué,
- W représente un atome d'oxygène ou un atome de soufre,
- $R_4$ et $R_5$, identiques ou différents, représentent un atome d'hydrogène, un atome d'halogène, un radical hydroxy, un radical alcoyle ou alcoxy renfermant de 1 à 3 atomes de carbone, un groupement trifluorométhyle, étant entendu que lorsque chacun des substituants $R_1$ représente un radical alcoyle, X et Y sont identiques ainsi que leurs sels d'addition avec les acides minéraux ou organiques, caractérisé en ce que l'on fait réagir un produit de formule (II) :

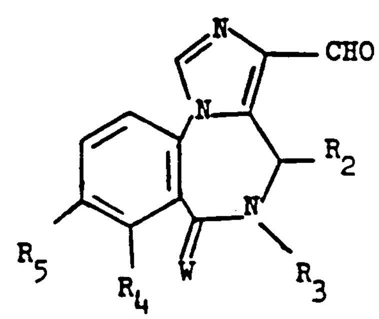

(II)

dans laquelle $R_2$, $R_3$, W, $R_4$ et $R_5$ ont la signification déjà indiquée, soit avec un produit de formule (III) :

$H\text{-}X\text{-}R_A$ (III)

dans laquelle X a la signification déjà indiquée et $R_A$ représente un radical alcoyle renfermant de 1 à 3 atomes de carbone, pour obtenir un produit de formule ($I_A$) :

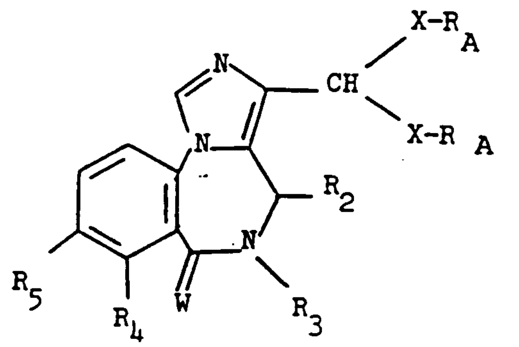

($I_A$)

que l'on peut salifier si désiré, soit avec un produit de formule (IV) :

$H\text{-}X\text{-}R_B$
$H\text{-}Y\text{-}R_B$ (IV)

dans laquelle X et Y ont la signification déjà indiquée et les substituants $R_B$ forment ensemble un pont alkylène renfermant de 2 à 5 atomes de carbone, pour obtenir le produit de formule ($I_B$) :

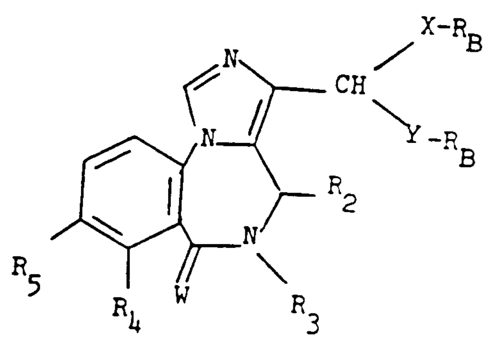

($I_B$)

que l'on peut salifier si désiré.

**2.** Procédé selon la revendication 1, caractérisé en ce que la réaction du produit de formule (II) avec le produit de formule (III) ou avec le produit de formule (IV) est effectuée au sein d'un solvant organique anhydre tel que le benzène ou le dichlorométhane.

**3.** Procédé selon la revendication 1 ou 2, carctérisé en ce que l'on utilise au départ un produit de formule (II) dans laquelle W représente un atome d'oxygène, $R_2$ et $R_5$ représentent un atome d'hydrogène, $R_4$ représente un atome d'halogène ou un groupement trifluorométhyle et $R_3$ a la signification indiquée à la revendication 1.

**4.** Procédé selon l'une quelconque des revendications 1 à 3, carctérisé en ce que l'on utilise au départ un produit de formule (II) dans laquelle $R_3$ représente un radical méthyle.

**5.** Procédé selon la revendication 1, caractérisé en ce que l'on met en oeuvre des produits de formule (II) choisis de manière telle que l'on prépare l'un quelconque des produits suivants :
- la 5,6- dihydro-3-(1,3-dioxalan-2-yl)-5-méthyl-imidazo [1,5-a] [1,4] benzodiazépine-6 (4H)-one.
- la 5,6-dihydro-3-(1,3-dithiolan-2-yl)-5-méthyl-imidazo [1,5-a] [1,4] benzodiazépine-6 (4H)-one.
- la 5,6-dihydro 3-(1,3-dioxolan-2-yl) 5-méthyl 7-trifluorométhyl imidazo [1,5-a] [1,4] benzodiazépine 6(4H)-one.

- la 5,6-dihydro 5-méthyl 3-(1,3-oxathiolan-2-yl) 7-trifluorométhyl imidazo [1,5-a] [1,4] benzodiazépine 6(4H)-one.
- la 5,6-dihydro 5-méthyl 3-(1,3-oxathiolan-2-yl) imidazo [1,5-a] [1,4] benzodiazépine 6(4H)-one.
- la 5,6-dihydro 7-fluoro 5-méthyl 3-(1,3-oxathiolan-2-yl) imidazo [1,5-a] [1,4] benzodiazépine 6(4H)-one.

6. Procédé de préparation de compositions pharmaceutiques, caractérisé en ce que l'on met à titre de principe actif l'un au moins des produits de formule générale (I) telle que définie à l'une quelconque des revendications 1 à 4 ou l'un au moins de leurs sels d'addition avec les acides pharmaceutiquement acceptables sous une forme destinée à cet usage.

7. Procédé de préparation de compositions pharmaceutiques, caractérisé en ce que l'on met à titre de principe actif l'un au moins des produits de formule générale (I) telle que définie à la revendication 5 ou l'un au moins de leurs sels d'addition avec les acides pharmaceutiquement acceptables sous une forme destinée à cet usage.

**Claims**

**Claims for the following Contracting States : AT, BE, CH, DE, DK, FR, GB, IT, LI, LU, NL, SE**

1. New imidazobenzodiazepines corresponding to general formula (I):

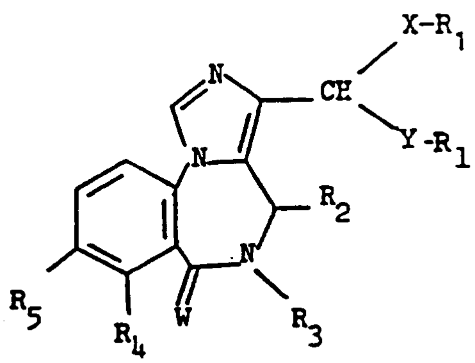

(I)

in which:
- the $R_1$ substituents represent an alkyl radical containing 1 to 3 carbon atoms or together form an alkylene bridge containing 2 to 5 carbon atoms,
- X and Y, identical or different, represent an oxygen atom or a sulphur atom,
- $R_2$ represents a hydrogen atom, an alkyl radical containing 1 to 3 carbon atoms,
- $R_3$ represents a hydrogen atom, an alkyl radical containing 1 to 5 carbon atoms, a phenyl group, a substituted phenyl group,
- W represents an oxygen atom or a sulphur atom,
- $R_4$ and $R_5$, identical or different, represent a hydrogen atom, a halogen atom, a hydroxy radical, an alkyl or alkoxy radical containing 1 to 3 carbon atoms, a trifluoromethyl group, it being understood that when each of the $R_1$ substituents represents an alkyl radical, X and Y are identical, as well as their addition salts with mineral or organic acids.

2. New imidazobenzodiazepines as defined by formula (I) of claim 1, characterized in that in the said formula (I), W represents an oxygen atom, $R_2$ and $R_5$ represent a hydrogen atom, $R_4$ represents a halogen atom or a trifluoromethyl group and X, Y, $R_1$ and $R_3$ have the meaning indicated in claim 1, as well as their addition salts with mineral or organic acids.

3. New imidazobendiazepines corresponding to formula (I), according to claim 1 or 2, characterized in that in the said formula (I), $R_3$ represents a methyl radical, $R_1$, Y, X, $R_2$, $R_4$, $R_5$ and W have the meaning already indicated, as well as their addition salts with mineral or organic acids.

4. 5,6-dihydro-3-(1,3-dioxalan-2-yl)-5-methyl-imidazo [1,5-a] [1,4] benzodiazepine-6(4H)-one.

**5.** 5,6-dihydro-3-(1,3-dithiolan-2-yl)-5-methyl-imidazo [1,5-a] [1,4] benzodiazepine-6(4H)-one.

**6.** 5,6-dihydro 3-(1,3-dioxolan-2-yl) 5-methyl 7-trifluoromethyl imidazo [1,5-a] [1,4] benzodiazepine 6(4H)-one.

**7.** 5,6-dihydro 5-methyl 3-(1,3-oxathiolan-2-yl) 7-trifluoromethyl imidazo [1,5a] [1,4] benzodiazepine 6(4H)-one.

**8.** 5,6-dihydro 5-methyl 3-(1,3-oxathiolan-2-yl) imidazo [1,5-a] [1,4] benzodiazepine 6(4H)-one.

**9.** 5,6-dihydro 7-fluoro 5-methyl 3-(1,3-oxathiolan-2-yl) imidazo [1,5a] [1,4] benzodiazepine 6(4H)-one.

**10.** Preparation process for new imidazobenzodiazepines as defined by formula (I) of claim 1, as well as their salts, characterized in that a product of formula (II):

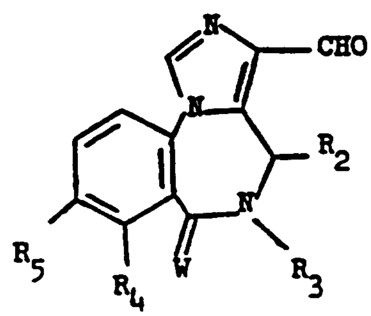

(II)

in which $R_2$, $R_3$, W, $R_4$ and $R_5$ have the meaning already indicated, is reacted either with a product of formula (III):

$H-X-R_A$ (III)

in which X has the meaning already indicated and $R_A$ represents an alkyl radical containing 1 to 3 carbon atoms, in order to obtain a product of formula ($I_A$):

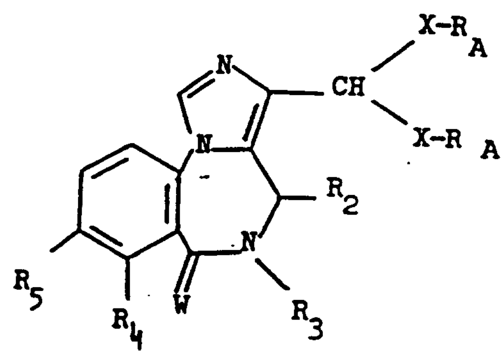

($I_A$)

which can be salified if desired, or with a product of formula (IV):

$H-X-R_B$
$H-Y-R_B$ (IV)

in which X and Y have the meaning already indicated and the $R_B$ substituents together form an alkylene bridge containing 2 to 5 carbon atoms, in order to obtain the product of formula ($I_B$):

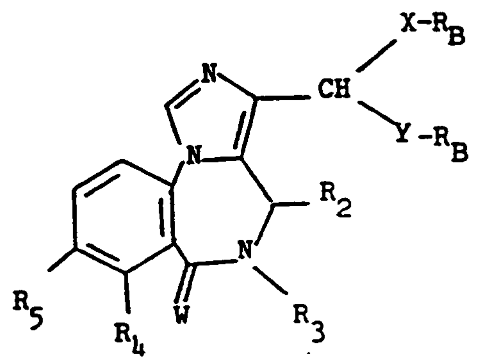

$(I_B)$

which can be salified if desired.

**11.** Process according to claim 10, characterized in that the reaction of the product of formula (II) with the product of formula (III) or with the product of formula (IV) is carried out in an anhydrous organic solvent such as benzene or dichloromethane.

**12.** Medicaments, characterized in that they are constituted by new imidazobenzodiazepines as defined by formula (I) of claim 1, as well as by their pharmaceutically acceptable salts.

**13.** Medicaments, characterized in that they are constituted by new imidazobenzodiazepines as defined in any one of claims 2 to 9, as well as by their pharmaceutically acceptable salts.

**14.** Pharmaceutical compositions, characterized in that they contain as active ingredient, at least one of the medicaments as defined in any one of claims 12 or 13.

**Claims for the following Contracting State : ES**

**1.** Process for preparing new imidazobenzodiazepines corresponding to general formula (I):

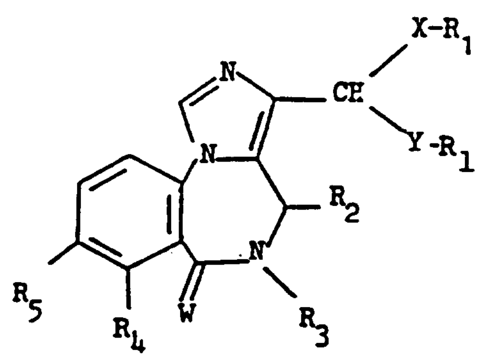

(I)

in which:

- the $R_1$ substituents represent an alkyl radical containing 1 to 3 carbon atoms or together form an alkylene bridge containing 2 to 5 carbon atoms,
- X and Y, identical or different, represent an oxygen atom or a sulphur atom,
- $R_2$ represents a hydrogen atom, an alkyl radical containing 1 to 3 carbon atoms,
- $R_3$ represents a hydrogen atom, an alkyl radical containing 1 to 5 carbon atoms, a phenyl group, a substituted phenyl group,
- W represents an oxygen atom or a sulphur atom,
- $R_4$ and $R_5$, identical or different, represent a hydrogen atom, a halogen atom, a hydroxy radical, an alkyl or alkoxy radical containing 1 to 3 carbon atoms, a trifluoromethyl group, it being understood that when each of the $R_1$ substituents represents an alkyl radical, X and Y are identical, as well as their addition salts with mineral or organic acids, characterized in that a

product of formula (II):

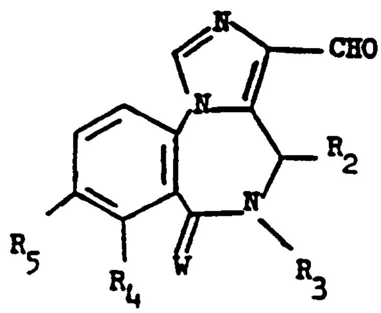

(II)

in which $R_2$, $R_3$, W, $R_4$ and $R_5$ have the meaning already indicated, is reacted either with a product of formula (III):

$H\text{-}X\text{-}R_A$ (III)

in which X has the meaning already indicated and $R_A$ represents an alkyl radical containing 1 to 3 carbon atoms, in order to obtain a product of formula ($I_A$):

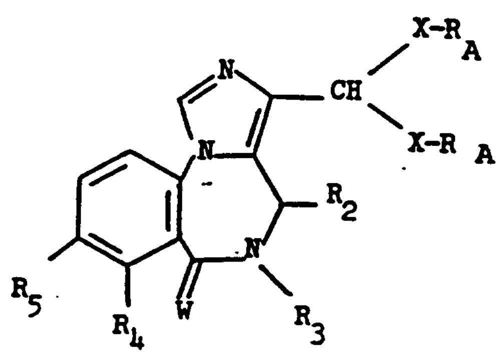

($I_A$)

which can be salified if desired, or with a product of formula (IV):

$H\text{-}X\text{-}R_B$
$H\text{-}Y\text{-}R_B$ (IV)

in which X and Y have the meaning already indicated and the $R_B$ substituents together form an alkylene bridge containing 2 to 5 carbon atoms, in order to obtain the product of formula ($I_B$):

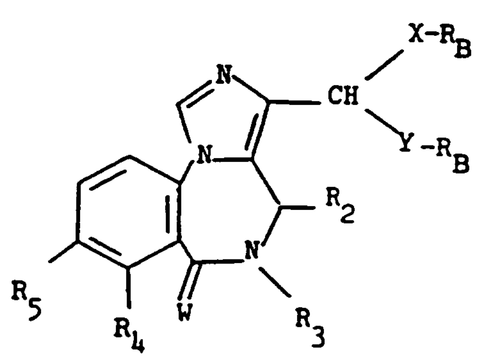

($I_B$)

which can be salified if desired.

**2.** Process according to claim 1, characterized in that the reaction of the product of formula (II) with the product of formula (III) or with the product of formula (IV) is carried out in an anhydrous organic solvent such as benzene or dichloromethane.

**3.** Process according to claim 1 or 2, characterized in that a product of formula (II) is used at the start in which W represents an oxygen atom, $R_2$ and $R_5$ represent a hydrogen atom, $R_4$ represents a halogen atom or a trifluoromethyl group and $R_3$ has the meaning indicated in claim 1.

**4.** Process according to any one of claims 1 to 3, characterized in that a product of formula (II) is used at the start in which $R_3$ represents a methyl radical.

**Claims for the following Contracting State : GR**

**1.** Process for preparing new imidazobenzodiazepines corresponding to general formula (I):

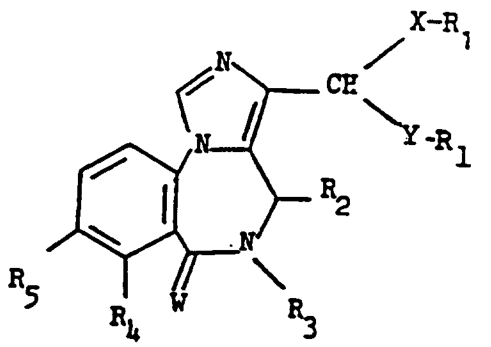

(I)

in which:

- the $R_1$ substituents represent an alkyl radical containing 1 to 3 carbon atoms or together form an alkylene bridge containing 2 to 5 carbon atoms,
- X and Y, identical or different, represent an oxygen atom or a sulphur atom,
- $R_2$ represents a hydrogen atom, an alkyl radical containing 1 to 3 carbon atoms,
- $R_3$ represents a hydrogen atom, an alkyl radical containing 1 to 5 carbon atoms, a phenyl group, a substituted phenyl group,
- W represents an oxygen atom or a sulphur atom,
- $R_4$ and $R_5$, identical or different, represent a hydrogen atom, a halogen atom, a hydroxy radical, an alkyl or alkoxy radical containing 1 to 3 carbon atoms, a trifluoromethyl group, it being understood that when each of the $R_1$ substituents represents an alkyl radical, X and Y are identical, as well as their addition salts with mineral or organic acids, characterized in that a product of formula (II):

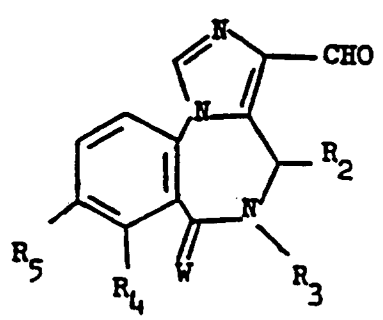

(II)

in which $R_2$, $R_3$, W, $R_4$ and $R_5$ have the meaning already indicated, is reacted either with a product of formula (III):

$H\text{-}X\text{-}R_A$ (III)

in which X has the meaning already indicated and $R_A$ represents an alkyl radical containing 1 to 3 carbon atoms, in order to obtain a product of formula ($I_A$):

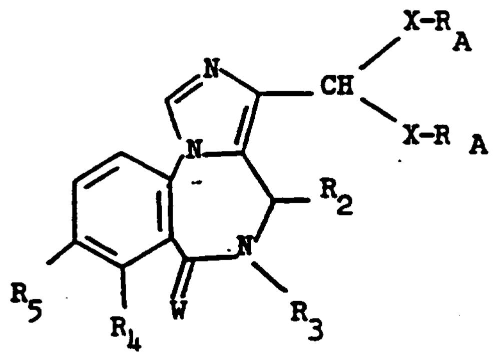

($I_A$)

which can be salified if desired, or with a product of formula (IV):

H-X-$R_B$
H-Y-$R_B$ (IV)

in which X and Y have the meaning already indicated and the $R_B$ substituents together form an alkylene bridge containing 2 to 5 carbon atoms, in order to obtain the product of formula ($I_B$):

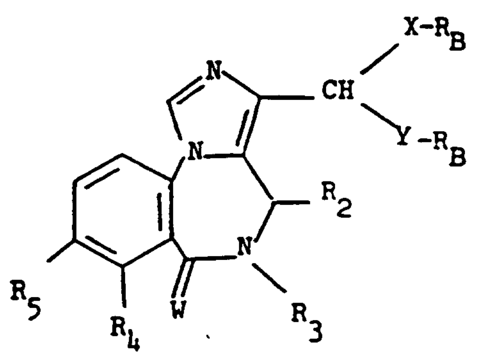

($I_B$)

which can be salified if desired.

**2.** Process according to claim 1, characterized in that the reaction of the product of formula (II) with the product of formula (III) or with the product of formula (IV) is carried out in an anhydrous organic solvent such as benzene or dichloromethane.

**3.** Process according to claim 1 or 2, characterized in that a product of formula (II) is used at the start in which W represents an oxygen atom, $R_2$ and $R_5$ represent a hydrogen atom, $R_4$ represents a halogen atom or a trifluoromethyl group and $R_3$ has the meaning indicated in claim 1.

**4.** Process according to any one of claims 1 to 3, characterized in that a product of formula (II) is used at the start in which $R_3$ represents a methyl radical.

**5.** Process according to claim 1, characterized in that products of formula (II) are used, chosen in such a way that any one of the following products are prepared:
- 5,6-dihydro-3-(1,3-dithiolan-2-yl)-5-methyl-imidazo [1,5-a] [1,4] benzodiazepine-6(4H)-one.
- 5,6-dihydro 3-(1,3-dioxolan-2-yl) 5-methyl 7-trifluoromethyl imidazo [1,5-a] [1,4] benzodiazepine 6-(4H)-one.

- 5,6-dihydro 5-methyl 3-(1,3-oxathiolan-2-yl) 7-trifluoromethyl imidazo [1,5-a] [1,4] benzodiazepine 6(4H)-one.
- 5,6-dihydro 5-methyl 3-(1,3-oxathiolan-2-yl) imidazo [1,5-a] [1,4] benzodiazepine 6(4H)-one.
- 5,6-dihydro 7-fluoro 5-methyl 3-(1,3-oxathiolan-2-yl) imidazo [1,5-a] [1,4] benzodiazepine 6(4H)-one.

**6.** Preparation process for pharmaceutical compositions, characterized in that at least one of the products of general formula (I) as defined in any one of claims 1 to 4 or at least one of their addition salts with pharmaceutically acceptable acids is used as active ingredient in a form intended for this use.

**7.** Preparation process for pharmaceutical compositions, characterized in that at least one of the products of general formula (I) as defined in claim 5 or at least one of their addition salts with pharmaceutically acceptable acids is used as active ingredient in a form intended for this use.

## Patentansprüche

## Patentansprüche für folgende Vertragsstaaten : AT, BE, CH, DE, DK, FR, GB, IT, LI, LU, NL, SE

**1.** Neue Imidazobenzodiazepine der allgemeinen Formel (I):

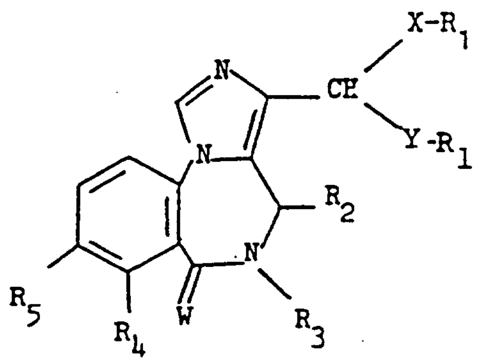

(I)

worin

- die Substituenten $R_1$ für einen Alkylrest mit 1 bis 3 Kohlenstoffatomen stehen oder zusammen eine Alkylenbrücke mit 2 bis 5 Kohlenstoffatomen bilden,
- X und Y, die gleich oder verschieden sind, für ein Sauerstoff- oder ein Schwefelatom stehen,
- $R_2$ für ein Wasserstoffatom, einen Alkylrest mit 1 bis 3 Kohlenstoffatomen steht,
- $R_3$ für ein Wasserstoffatom, einen Alkylrest mit 1 bis 5 Kohlenstoffatomen, eine Phenylgruppe, eine substituierte Phenylgruppe steht,
- W für ein Sauerstoff- oder ein Schwefelatom steht,
- $R_4$ und $R_5$, die gleich oder verschieden sind, für ein Wasserstoffatom, ein Halogenatom, eine Hydroxylgruppe, einen Alkyl- oder Alkoxyrest mit 1 bis 3 Kohlenstoffatomen, eine Trifluormethylgruppe stehen, mit der Maßgabe, daß wenn jeder der Substituenten $R_1$ für einen Alkylrest steht, X und Y gleich sind, sowie ihre Additionssalze mit mineralischen oder organischen Säuren.

**2.** Neue Imidazobenzodiazepine der Formel (I) nach Anspruch 1, dadurch **gekennzeichnet**, daß in der Formel (I) W für ein Sauerstoffatom steht, $R_2$ und $R_5$ für ein Wasserstoffatom stehen, $R_4$ für ein Halogenatom oder eine Trifluormethylgruppe steht, und X, Y, $R_1$ und $R_3$ wie für Anspruch 1 definiert sind, sowie ihre Additionssalze mit mineralischen oder organischen Säuren.

**3.** Neue Imidazobenzodiazepine der Formel (I) nach Anspruch 1 oder 2, dadurch **gekennzeichnet**, daß in der Formel (I) $R_3$ für eine Methylgruppe steht, $R_1$, Y, X, $R_2$, $R_5$ und W wie vorstehend definiert sind, sowie ihre Additionssalze mit mineralischen oder organischen Säuren.

**4.** 5,6-Dihydro-3-(1,3-dioxolan-2-yl)-5-methylimidazo[1,5-a] [1,4]benzodiazepin-6-(4H)-on.

**5.** 5,6-Dihydro-3-(1,3-dithiolan-2-yl)-5-methylimidazo [1,5-a] [1,4]benzodiazepin-6-(4H)-on.

**6.** 5,6-Dihydro-3-(1,3-dioxolan-2-yl)-5-methyl-7-trifluormethylimidazo[1,5-a] [1,4]benzodiazepin-6-(4H)-on.

**7.** 5,6-Dihydro-5-methyl-3-(1,3-oxathiolan-2-yl)-7-trifluormethylimidazo[1,5-a] [1,4]benzodiazepin-6-(4H)-on.

**8.** 5,6-Dihydro-5-methyl-3-(1,3-oxathiolan-2-yl)imidazo [1,5-a] [1,4]benzodiazepin-6-(4H)-on.

**9.** 5,6-Dihydro-7-fluor-5-methyl-3-(1,3-oxathiolan-2-yl)imidazo[1,5-a] [1,4]benzodiazepin-6-(4H)-on.

**10.** Verfahren zur Herstellung der neuen Imidazobenzodiazepine der Formel (I) nach Anspruch 1, sowie ihrer Salze, dadurch **gekennzeichnet**, daß man eine Verbindung der Formel (II)

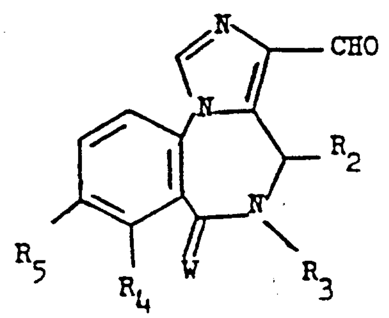

(II)

worin $R_2$, $R_3$, W, $R_4$ und $R_5$ wie vorstehend definiert sind, entweder mit einer Verbindung der Formel (III)

$H\text{-}X\text{-}R_A$ (III)

worin X wie vorstehend definiert ist, und $R_A$ für einen Alkylrest mit 1 bis 3 Kohlenstoffatomen steht, unter Erhalt einer Verbindung der Formel ($I_A$)

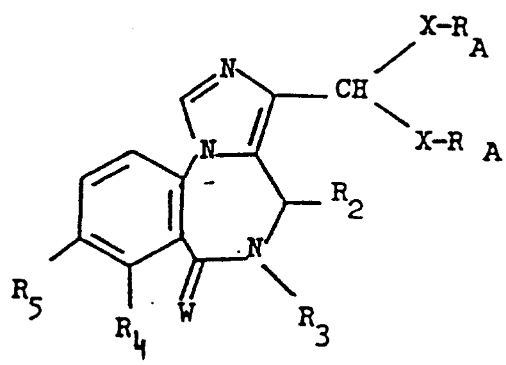

($I_A$)

die man gegebenenfalls in ihr Salz überführen kann, oder mit einer Verbindung der Formel (IV)

$H\text{-}X\text{-}R_B$
$H\text{-}Y\text{-}R_B$ (IV)

worin X und Y wie vorstehend definiert sind, und die Substituenten $R_B$ zusammen eine Alkylenbrücke mit 2 bis 5 Kohlenstoffatomen bilden, unter Erhalt der Verbindung der Formel ($I_B$)

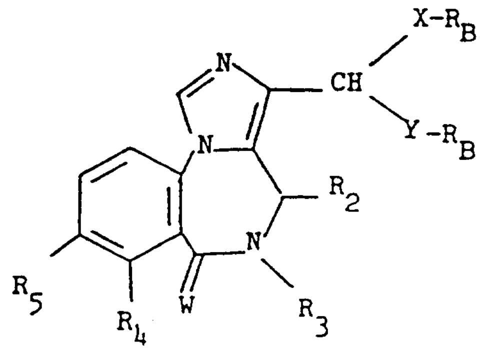

$(I_B)$

die man gegebenenfalls in ihr Salz überführen kann, umsetzt.

11. Verfahren nach Anspruch 10, dadurch **gekennzeichnet**, daß man die Umsetzung der Verbindung der Formel (II) mit der Verbindung der Formel (III) oder mit der Verbindung der Formel (IV) in einem organischen wasserfreien Lösungsmittel, wie Benzol oder Dichlormethan, durchführt.

12. Arzneimittel, dadurch **gekennzeichnet**, daß sie aus den neuen Imidazobenzodiazepinen der Formel (I) nach Anspruch 1, sowie ihren pharmazeutisch verträglichen Salzen bestehen.

13. Arzneimittel, dadurch **gekennzeichnet**, daß sie aus den neuen Imidazobenzodiazepinen nach einem der Ansprüche 2 bis 9, sowie ihren pharmazeutisch verträglichen Salzen bestehen.

14. Pharmazeutische Präparate, dadurch **gekennzeichnet**, daß sie als Wirkstoff mindestens eines der Arzneimittel nach einem der Ansprüche 12 oder 13 umfassen.

**Patentansprüche für folgenden Vertragsstaat : ES**

1. Verfahren zur Herstellung der neuen Imidazobenzodiazepine der allgemeinen Formel (I):

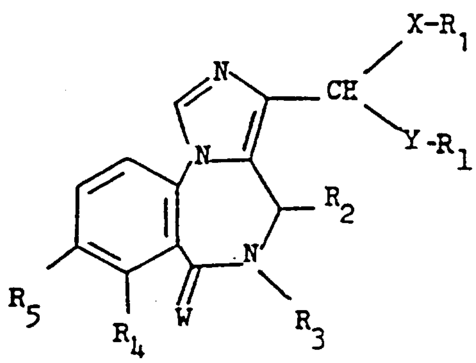

(I)

worin

- die Substituenten $R_1$ für einen Alkylrest mit 1 bis 3 Kohlenstoffatomen stehen oder zusammen eine Alkylenbrücke mit 2 bis 5 Kohlenstoffatomen bilden,
- X und Y, die gleich oder verschieden sind, für ein Sauerstoff- oder ein Schwefelatom stehen,
- $R_2$ für ein Wasserstoffatom, einen Alkylrest mit 1 bis 3 Kohlenstoffatomen steht,
- $R_3$ für ein Wasserstoffatom, einen Alkylrest mit 1 bis 5 Kohlenstoffatomen, eine Phenylgruppe, eine substituierte Phenylgruppe steht,
- W für ein Sauerstoff- oder ein Schwefelatom steht,
- $R_4$ und $R_5$, die gleich oder verschieden sind, für ein Wasserstoffatom, ein Halogenatom, eine Hydroxylgruppe, einen Alkyl- oder Alkoxyrest mit 1 bis 3 Kohlenstoffatomen, eine Trifluormethylgruppe stehen, mit der Maßgabe, daß wenn jeder der Substituenten $R_1$ für einen Alkylrest steht, X und Y gleich sind, sowie ihrer Additionssalze mit mineralischen oder organischen Säuren, dadurch **gekennzeichnet**, daß man eine Verbindung der Formel (II)

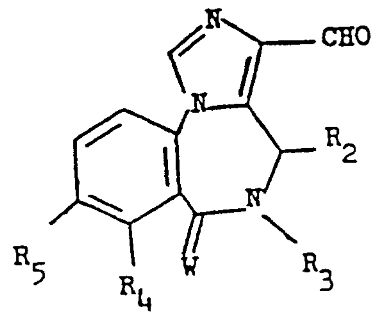

(II)

worin $R_2$, $R_3$, W, $R_4$ und $R_5$ wie vorstehend definiert sind, entweder mit einer Verbindung der Formel (III)

$H\text{-}X\text{-}R_A$ (III)

worin X wie vorstehend definiert ist, und $R_A$ für einen Alkylrest mit 1 bis 3 Kohlenstoffatomen steht, unter Erhalt einer Verbindung der Formel ($I_A$)

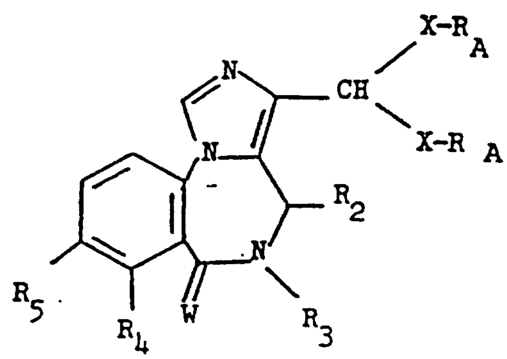

($I_A$)

die man gegebenenfalls in ihr Salz überführen kann, oder mit einer Verbindung der Formel (IV)

$H\text{-}X\text{-}R_B$
$H\text{-}Y\text{-}R_B$ (IV)

worin X und Y wie vorstehend definiert sind, und die Substituenten $R_B$ zusammen eine Alkylenbrücke mit 2 bis 5 Kohlenstoffatomen bilden, unter Erhalt der Verbindung der Formel ($I_B$)

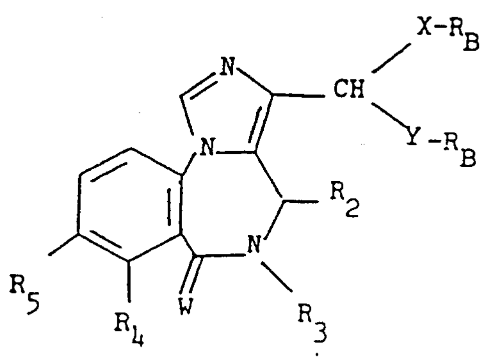

($I_B$)

die man gegebenenfalls in ihr Salz überführen kann, umsetzt.

2. Verfahren nach Anspruch 1, dadurch **gekennzeichnet**, daß man die Umsetzung der Verbindung der Formel (II) mit der Verbindung der Formel (III) oder mit der Verbindung der Formel (IV) in einem

organischen wasserfreien Lösungsmittel, wie Benzol oder Dichlormethan, durchführt.

3. Verfahren nach Anspruch 1 oder 2, dadurch **gekennzeichnet**, daß man als Ausgangsverbindung eine Verbindung der Formel (II) verwendet, worin W für ein Sauerstoffatom steht, $R_2$ und $R_5$ für ein Wasserstoffatom stehen, $R_4$ für ein Halogenatom oder eine Trifluormethylgruppe steht, und $R_3$ wie für Anspruch 1 definiert ist.

4. Verfahren nach einem der Ansprüche 1 bis 3, dadurch **gekennzeichnet**, daß man als Ausgangsverbindung eine Verbindung der Formel (II) verwendet, worin $R_3$ für eine Methylgruppe steht.

**Patentansprüche für folgenden Vertragsstaat : GR**

1. Verfahren zur Herstellung der neuen Imidazobenzodiazepine der allgemeinen Formel (I):

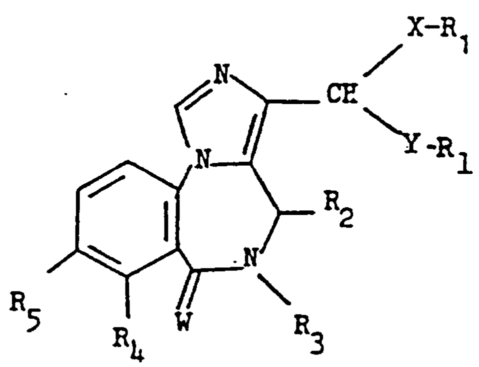

(I)

worin

- die Substituenten $R_1$ für einen Alkylrest mit 1 bis 3 Kohlenstoffatomen stehen oder zusammen eine Alkylenbrücke mit 2 bis 5 Kohlenstoffatomen bilden,
- X und Y, die gleich oder verschieden sind, für ein Sauerstoff- oder ein Schwefelatom stehen,
- $R_2$ für ein Wasserstoffatom, einen Alkylrest mit 1 bis 3 Kohlenstoffatomen steht,
- $R_3$ für ein Wasserstoffatom, einen Alkylrest mit 1 bis 5 Kohlenstoffatomen, eine Phenylgruppe, eine substituierte Phenylgruppe steht,
- W für ein Sauerstoff- oder ein Schwefelatom steht,
- $R_4$ und $R_5$, die gleich oder verschieden sind, für ein Wasserstoffatom, ein Halogenatom, eine Hydroxylgruppe, einen Alkyl- oder Alkoxyrest mit 1 bis 3 Kohlenstoffatomen, eine Trifluormethylgruppe stehen, mit der Maßgabe, daß wenn jeder der Substituenten $R_1$ für einen Alkylrest steht, X und Y gleich sind, sowie ihrer Additionssalze mit mineralischen oder organischen Säuren, dadurch **gekennzeichnet**, daß man eine Verbindung der Formel (II)

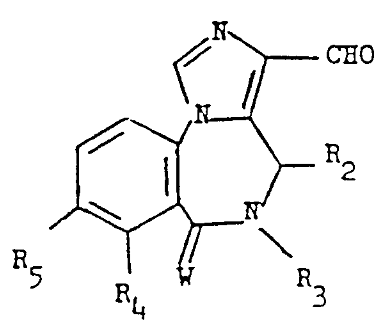

(II)

worin $R_2$, $R_3$, W, $R_4$ und $R_5$ wie vorstehend definiert sind, entweder mit einer Verbindung der Formel (III)

$H\text{-}X\text{-}R_A$ (III)

worin X wie vorstehend definiert ist, und $R_A$ für einen Alkylrest mit 1 bis 3 Kohlenstoffatomen steht, unter Erhalt einer Verbindung der Formel ($I_A$)

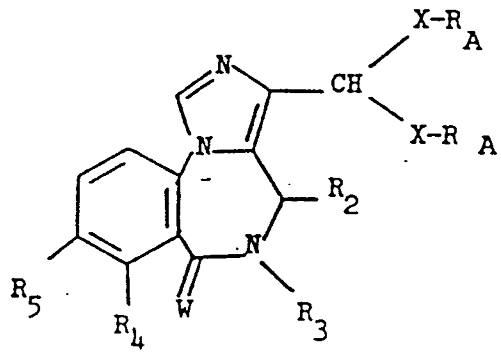

($I_A$)

die man gegebenenfalls in ihr Salz überführen kann, oder mit einer Verbindung der Formel (IV)

H-X-$R_B$
H-Y-$R_B$ (IV)

worin X und Y wie vorstehend definiert sind, und die Substituenten $R_B$ zusammen eine Alkylenbrücke mit 2 bis 5 Kohlenstoffatomen bilden, unter Erhalt der Verbindung der Formel ($I_B$)

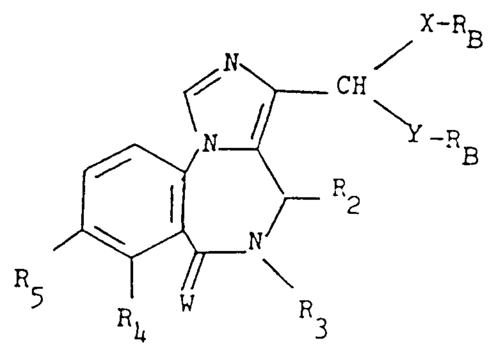

($I_B$)

die man gegebenenfalls in ihr Salz überführen kann, umsetzt.

2. Verfahren nach Anspruch 1, dadurch **gekennzeichnet**, daß man die Umsetzung der Verbindung der Formel (II) mit der Verbindung der Formel (III) oder mit der Verbindung der Formel (IV) in einem organischen wasserfreien Lösungsmittel, wie Benzol oder Dichlormethan, durchführt.

3. Verfahren nach Anspruch 1 oder 2, dadurch **gekennzeichnet**, daß man als Ausgangsverbindung eine Verbindung der Formel (II) verwendet, worin W für ein Sauerstoffatom steht, $R_2$ und $R_5$ für ein Wasserstoffatom stehen, $R_4$ für ein Halogenatom oder eine Trifluormethylgruppe steht, und $R_3$ wie für Anspruch 1 definiert ist.

4. Verfahren nach einem der Ansprüche 1 bis 3, dadurch **gekennzeichnet**, daß man als Ausgangsverbindung eine Verbindung der Formel (II) verwendet, worin $R_3$ für eine Methylgruppe steht.

5. Verfahren nach Anspruch 1, dadurch **gekennzeichnet**, daß man Verbindungen der Formel (II) verwendet, die so ausgewählt sind, daß man eine der nachstehenden Verbindungen herstellt:
   - 5,6-Dihydro-3-(1,3-dioxolan-2-yl)-5-methylimidazo[1,5-a] [1,4]benzodiazepin-6-(4H)-on.
   - 5,6-Dihydro-3-(1,3-dithiolan-2-yl)-5-methylimidazo[1,5-a] [1,4]benzodiazepin-6-(4H)-on
   - 5,6-Dihydro-3-(1,3-dioxolan-2-yl)-5-methyl-7-trifluormethylimidazo[1,5-a] [1,4]benzodiazepin-6-(4H)-on.

- 5,6-Dihydro-5-methyl-3-(1,3-oxathiolan-2-yl)-7-trifluormethylimidazo[1,5-a] [1,4]benzodiazepin-6-(4H)-on.
- 5,6-Dihydro-5-methyl-3-(1,3-oxathiolan-2-yl)imidazo [1,5-a] [1,4]benzodiazepin-6-(4H)-on.
- 5,6-Dihydro-7-fluor-5-methyl-3-(1,3-oxathiolan-2-yl)imidazo[1,5-a] [1,4]benzodiazepin-6-(4H)-on.

**6.** Verfahren zur Herstellung von pharmazeutischen Präparaten, dadurch **gekennzeichnet**, daß man als Wirkstoff mindestens eine der Verbindungen der allgemeinen Formel (I) nach einem der Ansprüche 1 bis 4 oder mindestens eines ihrer Additionssalze mit pharmazeutisch verträglichen Säuren in einer für diese Verwendung geeigneten Form einsetzt.

**7.** Verfahren zur Herstellung von pharmazeutischen Präparaten, dadurch **gekennzeichnet**, daß man als Wirkstoff mindestens eine der Verbindungen der allgemeinen Formel (I) nach Anspruch 5 oder mindestens eines ihrer Additionssalze mit pharmazeutisch verträglichen Säuren in einer für diese Verwendung geeigneten Form einsetzt.